# EUROPEAN PATENT SPECIFICATION

(11) **EP 2 936 159 B1**
(45) Date of publication and mention of the grant of the patent: **13.06.2018**
(21) Application number: 13811890.6
(22) Date of filing: 18.12.2013
(51) Int. Cl.: G01N 33/68

(54) **CHARACTERIZATION OF THERMOSTABLE DNA POLYMERASE**
CHARAKTERISIERUNG VON THERMOSTABILER DNA-POLYMERASE
CARACTÉRISATION DE L'ADN POLYMÉRASE THERMOSTABLE

(30) Priority: 20.12.2012 US 201261740162 P
(43) Date of publication of application: 28.10.2015
(73) Proprietor: Roche Diagnostics GmbH, 68305 Mannheim (DE); F. Hoffmann-La Roche AG, 4070 Basel (CH)
(72) Inventor: JUNG, Laura, Oakland, California 94619 (US)
(74) Representative: Schwarz, Ralf
(86) International application number: PCT/EP2013/077015
(87) International publication number: WO 2014/095951

(56) References cited:
- SUNG CHAN KIM ET AL: "Substrate and functional diversity of lysine acetylation revealed by a proteomics survey", MOLECULAR CELL, CELL PRESS, CAMBRIDGE, MA, US, vol. 23, no. 4, 1 August 2006 (2006-08-01) , pages 607-618, XP002658474, ISSN: 1097-2765
- Chan Sung ET AL: "Molecular Cell, Volume 23 Supplemental Data Substrate and Functional Diversity of Lysine Acetylation Revealed by a Proteomics Survey", , 1 August 2006 (2006-08-01), XP055107461, Retrieved from the Internet: URL:http://www.sciencedirect.com/science/a rticle/pii/S1097276506004540 [retrieved on 2014-03-13]
- CHEN YUE ET AL: "Lysine propionylation and butyrylation are novel post-translational modifications in histones", MOLECULAR & CELLULAR PROTEOMICS, AMERICAN SOCIETY FOR BIOCHEMISTRY AND MOLECULAR BIOLOGY, vol. 6, no. 5, 1 May 2007 (2007-05-01), pages 812-819, XP002522297, ISSN: 1535-9476, DOI: 10.1074/MCP.M700021-MCP200
- IDA CHIARA GUERRERA ET AL: "Application of Mass Spectrometry in Proteomics", BIOSCIENCE REPORTS, KLUWER ACADEMIC PUBLISHERS-PLENUM PUBLISHERS, NE, vol. 25, no. 1-2, 1 February 2005 (2005-02-01), pages 71-93, XP019274416, ISSN: 1573-4935
- FORNÉ I ET AL: "Quantifying histone modifications using mass spectrometry (Prot 51)", INTERNET CITATION, 20 January 2012 (2012-01-20), pages 1-14, XP002711276, Retrieved from the Internet: URL:http://www.epigenesys.eu/index.php/en/ protcols/histone-modifications-antibodies/ 251-quantifying-histone-modifications-usin g-mass-spectrometry [retrieved on 2013-08-13]
- YINGMING ZHAO ET AL: "Modification-specific proteomics: Strategies for characterization of post-translational modifications using enrichment techniques", PROTEOMICS, vol. 9, no. 20, 1 October 2009 (2009-10-01), pages 4632-4641, XP55107474, ISSN: 1615-9853, DOI: 10.1002/pmic.200900398
- ZEE B M ET AL: "Quantitative Proteomic Approaches to Studying Histone Modifications", CURRENT CHEMICAL GENOMICS, BENTHAM OPEN, NL, vol. 5, no. suppl. 1-M7, 1 January 2011 (2011-01-01), pages 106-114, XP002711277, ISSN: 1875-3973, DOI: 10.2174/1875397301005010106 [retrieved on 2011-08-22]
- KANGLING ZHANG ET AL: "Differentiation between peptides containing acetylated or tri-methylated lysines by mass spectrometry: An application for determining lysine 9 acetylation and methylation of histone H3", PROTEOMICS, vol. 4, no. 1, 1 January 2004 (2004-01-01) , pages 1-10, XP55107371, ISSN: 1615-9853, DOI: 10.1002/pmic.200300503

### SEQUENCE LISTING

<110> F. Hoffmann-La Roche AG Roche Diagnostics GmbH
<120> Characterization of Thermostable DNA Polymerase
<130> 31294 WO-HS
<140> US Not yet assigned
   <141> Not yet assigned
<150> US 61/740,162
   <151> 2012-12-20
<160> 7
<170> FastSEQ for Windows Version 4.0
<210> 1
   <211> 5
   <212> PRT
   <213> Artificial Sequence
<220>
   <223> synthetic Gold DNA polymerase trypsin protease digest peptide with single lysine, amino acids 705-709
<400> 1
   Ala Trp Ile Glu Lys
    1               5
<210> 2
   <211> 11
   <212> PRT
   <213> Artificial Sequence
<220>
   <223> synthetic Gold DNA polymerase trypsin protease digest peptide with single lysine, amino acids 705-715
<220>
   <221> MOD_RES
   <222> (5)...(5)
   <223> may be citraconylated Lys
<400> 2
   Ala Trp Ile Glu Lys Thr Leu Glu Glu Gly Arg
   1 5 10
<210> 3
   <211> 13
   <212> PRT
   <213> Artificial Sequence
<220>
   <223> synthetic Gold DNA polymerase trypsin protease digest peptide with single lysine, amino acids 493-505
<400> 3
   Val Leu Phe Asp Glu Leu Gly Leu Pro Ala Ile Gly Lys
   1 5 10
<210> 4
   <211> 16
   <212> PRT
   <213> Artificial Sequence
<220>
   <223> synthetic Gold DNA polymerase trypsin protease digest peptide with multiple lysines, amino acids 493-508
<220>
   <221> MOD_RES
   <222> (13)...(13)
   <223> may be citraconylated Lys
<400> 4
   Val Leu Phe Asp Glu Leu Gly Leu Pro Ala Ile Gly Lys Thr Glu Lys
   1 5 10 15
<210> 5
   <211> 19
   <212> PRT
   <213> Artificial Sequence
<220>
   <223> synthetic Gold DNA polymerase trypsin protease digest peptide with multiple lysines, amino acids 493-511
<220>
   <221> MOD_RES
   <222> (13)...(13)
   <223> may be citraconylated Lys
<220>
   <221> MOD_RES
   <222> (16)...(16)
   <223> may be citraconylated Lys
<400> 5
   Val Leu Phe Asp Glu Leu Gly Leu Pro Ala Ile Gly Lys Thr Glu Lys
    1               5                  10                  15
   Thr Gly Lys
<210> 6
   <211> 6
   <212> PRT
   <213> Artificial Sequence
<220>
   <223> synthetic Gold DNA polymerase trypsin protease digest peptide with multiple lysines, amino acids 506-511
<220>
   <221> MOD_RES
   <222> (3)...(3)
   <223> may be citraconylated Lys
<400> 6
   Thr Glu Lys Thr Gly Lys
    1               5
<210> 7
   <211> 7
   <212> PRT
   <213> Artificial Sequence
<220>
   <223> synthetic Gold DNA polymerase trypsin protease digest peptide with multiple lysines, amino acids 506-512
<220>
   <221> MOD_RES
   <222> (3)...(3)
   <223> may be citraconylated Lys
<220>
   <221> MOD_RES
   <222> (6)...(6)
   <223> may be citraconylated Lys
<400> 7
   Thr Glu Lys Thr Gly Lys Arg
    1               5

## Description

### BACKGROUND OF THE INVENTION

The polymerase chain reaction (PCR) process for amplifying nucleic acid sequences is well known in the art and disclosed in, for example, U.S. Pat. Nos. 4,683,202; 4,683,195; and 4,965,188. In each cycle of a PCR amplification, a double-stranded target sequence is denatured, primers are annealed to each strand of the denatured target sequence, and the primers are extended (elongated) by the action of a DNA polymerase. These steps can be summarized as denaturing, annealing and elongation step, respectively. Under the elevated temperatures used in a typical PCR, the primers hybridize only to the intended target sequence. However, amplification reaction mixtures are typically assembled at room temperature, well below the hybridization temperature of the primers. Under such less stringent conditions the primers may bind to only partially complementary sequences or other primers and initiate the synthesis of undesired extension products. These undesired extension products are amplified along with the target sequence and this amplification competes with the amplification of the desired target sequence decreasing the efficiency of specific amplification. Several methods are known in the art to increase efficiency and decrease unspecific amplification during PCR reactions. For instance, hot-start methods were initially carried out by manually opening the reaction tube after the initial high temperature incubation step and adding the missing reagents (e.g. DNA polymerase). This method was further improved by the development of chemically modified DNA polymerase. Chemically modified DNA polymerases are reversible inactivated polymerases, which are inactivated by covalent modifications, which are located at the active site of the enzyme or which cause a conformational change rendering the enzyme inactive. The chemical modification can be reversed by heat thereby transforming the enzyme into its active form. Therefore, a preassembled reaction mix containing the DNA polymerase in its inactive form can be directly used for amplification, without the extra step of adding the enzyme after the first high temperature incubation step. Chemically modified DNA polymerases and methods of modifying the same are described in US Pat. Nos. 5,773,258 and 5,677,152.

High quality DNA polymerases are useful for a variety of applications such as highly complex diagnostic tests that require high efficiency and specificity of the PCR amplification performed.

In the art methods of characterizing proteins, in particular histones, are known utilizing chemically modified lysine residues, in particular acetylated lysine residues, in mass spectrometry applications.

Kim SC et al. (Mol Cell (2006), 23(4): 607-618 and Supplemental Data) disclose methods for determining the number and site of covalent lysine modifications in lysine-acetylated proteins using MS/MS data of tryptic digests which are compared with unmodified standard peptides.

Chen Y et al. (Mol Cell Proteomics (2007), 6(5):812-819) disclose lysine propionylation and butyrylation as two novel in vivo lysine modifications in histones catalyzed by two previously known acetyltransferases, p300 and CREB-binding protein using peptide mapping by mass spectrometry.

Guerrera ID et al. (Biosci Rep (2005), 25(1-2):71-93) disclose principles of mass spectrometry and instrumentation that may be used in proteomics experiments, such as high-throughput identification of proteins from highly complex mixtures including accurate mass measurement of peptides derived from total proteome digests and multidimensional peptide separations coupled with mass spectrometry.

Zhao Y et al. (Proteomics (2009), 9(20):4632-4641) disclose methods for characterization of post-translational modifications such as phosphorylation, ubiquitination, and lysine acetylation using enrichment techniques combined with advanced MS/MS methods and computational data analysis.

Forné et al. ("Quantifying histone modifications using mass spectrometry (Prot 51)" (20 Jan 2012), Internet Citation on www.epigenesys.eu: 1-14) discloses methods for quantifying histone modifications using mass spectrometry, wherein lysines are chemically blocked by acylation before trypsin digestion and MALDI-TOF mass spectrometry.

Zee BM et al. (Curr Chem Genomics (2011), 5(Suppl. 1): 106-14) discloses methods for analyzing histone post-translational modifications using mass spectrometry, wherein lysines are chemically blocked by acylation before trypsin digestion and MS/MS analysis.

Zhang K et al. (Proteomics (2004), 4(1):1-10) discloses methods for differentiation between histone peptides containing acetylated or tri-methylated lysines by mass spectrometry.

### BRIEF SUMMARY OF THE INVENTION

The present invention provides, *inter alia*, methods of characterizing DNA polymerases using mass spectrometry. In particular, the methods provided herein can be used to detect chemical lysine modifications in a DNA polymerase thereby characterizing the activity level of said DNA polymerase.

In one aspect, a method of detecting the number of covalent lysine modifications in a lysine-modified DNA polymerase is provided. The method includes (i) detecting the mass of the lysine-modified DNA polymerase at a modification stabilizing pH of at least 8; and (ii) calculating the number of lysine modifications in the lysine-modified DNA polymerase by comparing the mass of the lysine-modified DNA polymerase to a polymerase without lysine modifications, wherein the number of lysine modifications is the difference of the mass of the lysine-modified DNA polymerase and the polymerase without lysine modifications divided by the mass of the lysine modification, thereby detecting the number of lysine modifications in the DNA polymerase polymerase, wherein said covalent lysine modification is a citraconyl modification, an aconitylated modification or a 2,3-dimethylmaleated modification.

In some embodiments, the mass of the lysine-modified DNA polymerase is a computer readable mass. In some embodiments, the mass of the polymerase without lysine modifications is constructed in silico, thereby forming a computer readable standard mass. In some embodiments, the calculating is performed on a computer.

In some embodiments, the DNA polymerase is substantially free of non-ionic detergent immediately prior to and during the detecting of step (i).

In some embodiments, the method further includes prior to the detecting of step (i), fragmenting the lysine-modified DNA polymerase with an enzyme that fragments the polymerase at known sites within the polymerase. In some embodiments, the enzyme is trypsin. In some embodiments, the method includes further comprises detecting the mass-to-charge ratio of fragments of the lysine-modified DNA polymerase and comparing the mass of fragments of the lysine-modified DNA polymerase to the mass of potential fragments of the polymerase without lysine modifications, thereby determining the location of lysine modifications at particular lysines in the polymerase.

In some embodiments, the method further includes detecting the relative amount of fragments of the lysine-modified DNA polymerase and optionally comparing the relative amount of the fragments of the lysine-modified DNA polymerase to a predicted relative amount of potential fragments of the polymerase without lysine modifications, thereby determining the relative level of lysine modifications at particular lysines in the polymerase.

In some embodiments, the location of lysine modification corresponds to amino acid position 540, 663, or 738 of Taq GOLD DNA polymerase. In some embodiments, the location of lysine modification corresponds to amino acid position 542, 665, or 740 of a Z05 GOLD DNA polymerase.

In some embodiments, the method provided herein includes detecting the relative amount of modification at the amino acid position, wherein the relative amount of modification is the amount of modified lysine residue at the position divided by the sum of the amount of modified and unmodified lysine residue at the position, thereby detecting the relative amount of modification at the position.

### BRIEF DESCRIPTION OF THE DRAWINGS

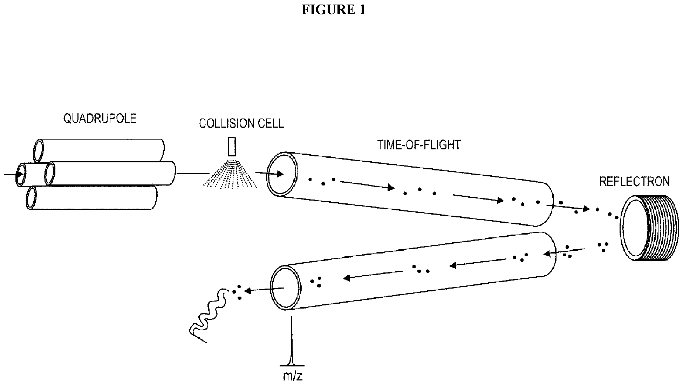

**Figure 1****.** Diagram of the quadrupole time-of-flight mass analyzer (from Siuzdak, G, "The Expanding Role of Mass Spectrometry in Biotechnology").
**Figure 2****.** Intact analysis of delta Z05: (A) Total ion chromatogram; (B) mass spectrum; (C) deconvoluted mass. The theoretical mass of delta Z05 is 61,076.04. The mass obtained by deconvolution of the mass spectrum was 60,874.91, which is consistent with the loss of the N-terminal methionine and alanine residues, within 18ppm (1.11amu).
**Figure 3****.** High pH intact analysis was used to determine a global distribution of 10-19 citraconyl modifications on delta Z05 GOLD. Each peak represents the mass of delta Z05 plus the designated number of citraconyl modifications.

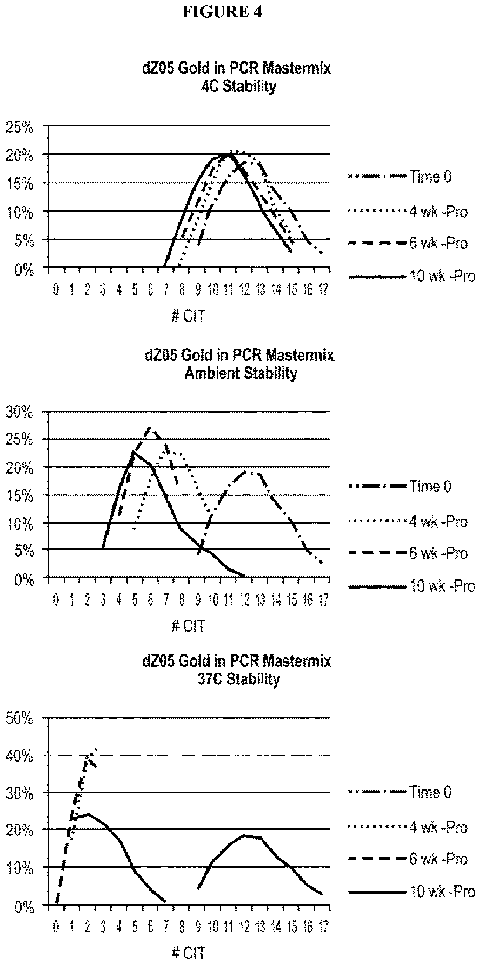

**Figure 4****.** Analysis of a PCR master mix modified to be compatible with LCMS. The enzyme concentration was increased and the salts, dNTPs, UNG and DMSO were excluded. Analysis of this master mix under accelerated storage conditions indicated a significant loss of the citraconyl modifications at elevated temperatures. Storage at 4 °C showed a net loss of two modifications after four months.
**Figure 5****.** Map of lysine residues in Taq GOLD and Z05 GOLD with the percentage of citraconyl modifications.
**Figure 6****.** Comparison of lysine residues in the DNA polymerase domains of Taq GOLD and Z05 GOLD. The Z05 GOLD has a net of five additional lysines which are all modified to some extent. The K540, K663 and K738 in Taq and their equivalent in Z05 are all heavily modified. A molecular model of Taq lacking the exonuclease domain (front view) indicates that they are in key positions.

### DETAILED DESCRIPTION OF THE INVENTION

### I. Definitions

The abbreviations used herein have their conventional meaning within the chemical and biological arts.

"Polymerase" refers to an enzyme that performs template-directed synthesis of polynucleotides. DNA polymerase can add free nucleotides only to the 3' end of the newly forming strand. This results in elongation of the newly forming strand in a 5'-3' direction. No known DNA polymerase is able to begin a new chain (de novo). DNA polymerase can add a nucleotide only on to a pre-existing 3'-OH group, and, therefore, needs a primer at which it can add the first nucleotide. Primers can comprise, for example, RNA and/or DNA bases, as well as non-naturally-occurring bases. The directionality of the newly forming strand (the daughter strand) is opposite to the direction in which DNA polymerase moves along the template strand. Non-limiting examples of polymerases include prokaryotic DNA polymerases (e.g. Pol I, Pol II, Pol III, Pol IV and Pol V), eukaryotic DNA polymerase, telomerase, reverse transcriptase and RNA polymerase. Reverse transcriptase is an RNA-dependent DNA polymerase which synthesizes DNA from an RNA template. The reverse transcriptase family contains both DNA polymerase functionality and RNase H functionality, which degrades RNA base-paired to DNA. RNA polymerase, is an enzyme that synthesizes RNA using DNA as a template during the process of gene transcription. RNA polymerase polymerizes ribonucleotides at the 3'end of an RNA transcript.

The term "thermostable polymerase" refers to an enzyme that is stable to heat, is heat resistant, and retains sufficient activity to effect subsequent polynucleotide extension reactions and does not become irreversibly denatured (inactivated) when subjected to the elevated temperatures for the time necessary to effect denaturation of double-stranded nucleic acids. The heating conditions necessary for nucleic acid denaturation are well known in the art and are exemplified in, *e.g.*, U.S. Patent Nos. 4,683,202, 4,683,195, and 4,965,188. As used herein, a thermostable polymerase is suitable for use in a temperature cycling reaction such as the polymerase chain reaction ("PCR"). Irreversible denaturation for purposes herein refers to permanent and complete loss of enzymatic activity. For a thermostable polymerase, enzymatic activity refers to the catalysis of the combination of the nucleotides in the proper manner to form polynucleotide extension products that are complementary to a template nucleic acid strand. Thermostable DNA polymerases from thermophilic bacteria include, *e.g*., DNA polymerases from *Thermotoga maritima*, *Therms aquaticus*, *Therms thermophilus*, *Thermus flavus, Thermus filiformis*, *Thermus* species sps 17, *Thermus* species Z05, *Thermus caldophilus*, *Bacillus caldotenax*, *Thermotoga neopolitana*, and *Thermosipho africanus*.

A "chemically modified DNA polymerase" as provided herein refers to a DNA polymerase with at least one covalently attached modification rendering the DNA polymerase at least substantially inactive. In some embodiments, the covalent modification is attached only to a particular kind of amino acid of the DNA polymerase (for example, to lysines only, or to arginines only, etc.). In some embodiments, the modification only occurs at a known set of amino acids (e.g., only on lysines and arginines, etc.). In some embodiments, the modifications can only occur at specific sites (e.g., at specific subsequences) in the polymerase. In some embodiments, the modification is attached to a lysine of the DNA polymerase. Where the modification is attached to a lysine, the DNA polymerase is referred to as "lysine-modified" DNA polymerase.

The terms "chemically modified" and "reversible inactivated" are used interchangeably throughout and in keeping with the plain ordinary usage of those terms. Thus, the term "reversibly inactive" or "reversibly inactivated" as provided herein refers to the reversible loss of all, or nearly all, of a DNA polymerase activity. The modifications are "reversible" because the modifications can be removed under conditions (e.g., heat) to result in a substantially active enzyme.

The term "modifier agent" as provided herein refers to a chemical compound capable of reacting with an amino acid of a DNA polymerase thereby forming a covalent amino acid (e.g. lysine modification).

A "modification-stabilizing pH" is a pH at which the chemical modification (e.g. covalent lysine modification) of a DNA polymerase remains substantially stable (attached to the polymerase). For example, a modification-stabilizing pH allows the modifications remain on the polymerase the pH during the process of liquid chromatography (LC) separation and subsequent quadrupole time-of-flight mass spectrometry (Q-TOF MS) analysis of the modified polymerase. In some cases, dicarboxylic acid anhydride modifications, as described herein, or unstable at lower pH ranges. Thus, a pH of 8 or above is a modification-stabilizing pH for such dicarboxylic acid anhydride modifications.

When referring to a position in a DNA polymerase, an amino acid "corresponding" to a location in another sequence is based on the convention of numbering according to nucleotide or amino acid position number and then aligning the sequences in a manner that maximizes the percentage of sequence identity. An amino acid "corresponding to position [X] of [specific sequence]" refers to an amino acid in a polypeptide of interest that aligns with the equivalent amino acid of a specified sequence. Generally, as described herein, the amino acid corresponding to a position of a polymerase can be determined using an alignment algorithm such as BLAST as described below. Because not all positions within a given "corresponding region" need be identical, non-matching positions within a corresponding region may be regarded as "corresponding positions." Accordingly, as used herein, referral to an "amino acid position corresponding to amino acid position [X]" of a specified DNA polymerase refers to equivalent positions, based on alignment, in other DNA polymerases and structural homologues and families.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters are commonly used, or alternative parameters can be designated. The sequence comparison algorithm then calculates the percent sequence identities or similarities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, for example, by the local homology algorithm of Smith and Waterman (Adv. Appl. Math. 2:482, 1970), by the homology alignment algorithm of Needleman and Wunsch (J. Mol. Biol. 48:443, 1970), by the search for similarity method of Pearson and Lipman (Proc. Natl. Acad. Sci. USA 85:2444, 1988), by computerized implementations of these algorithms (*e.g*., GAP, BESTFIT, FASTA, and TFASTA in the Wisconsin Genetics Software Package, Genetics Computer Group, 575 Science Dr., Madison, Wis.), or by manual alignment and visual inspection (*see*, *e.g.*, Ausubel et al., Current Protocols in Molecular Biology (1995 supplement)).

Examples of an algorithm that is suitable for determining percent sequence identity and sequence similarity are the BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*., *supra*)*.* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (*see* Henikoff and Henikoff, Proc. Natl. Acad. Sci. USA 89:10915, 1989) alignments (B) of 50, expectation (E) of 10, M=5, N=-4, and a comparison of both strands.

The BLAST algorithm also performs a statistical analysis of the similarity between two sequences (*see*, *e.g*., Karlin and Altschul, Proc. Natl. Acad. Sci. USA 90:5873-87, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, typically less than about 0.01, and more typically less than about 0.001.

### II. Methods of Detecting Chemical Modifications in DNA Polymerases

Methods of detecting the quantity and/or location and quantity of chemical modifications (e.g. lysine modifications) in a DNA polymerase are provided. In some embodiments, the quality and quantity of modifications is useful to monitor and control the quality of thermostable DNA polymerases during production, storage and use of the enzyme. Further, the methods are useful for characterizing newly designed chemical modifications that inactivate a DNA polymerase.

In one aspect, a method of detecting the number of covalent lysine modifications in a lysine-modified DNA polymerase is provided. However, it should be noted that the methods described herein can be applied to assessing other chemical modifications of polymerases or other proteins, so long as the possible sites of modification in the polymerase or other protein, as well as the mas of the modification, is predictable. In some embodiments, the method includes (i) detecting the mass of the lysine-modified DNA polymerase at a modification stabilizing pH and (ii) calculating the number of lysine modifications in the lysine-modified DNA polymerase by comparing the mass of the lysine-modified DNA polymerase to a polymerase without lysine modifications, wherein the number of lysine modifications is the difference of the mass of the lysine-modified DNA polymerase and the polymerase without lysine modifications divided by the mass of the lysine modification, thereby detecting the number of lysine modifications in the DNA polymerase.

As described above, chemically modified thermostable DNA polymerases such as lysine-modified DNA polymerases are used in PCR applications (e.g. Hot Start PCR) that require high specificity (decreased nonspecific product amplification) and increased sensitivity and yield (low concentration of template DNA). A chemically modified thermostable DNA polymerase is substantially inactive at temperatures below or equivalent to the primer annealing temperature. Thus, a chemically modified DNA polymerase is not active at non-stringent conditions and synthesis of undesired extension products caused by unintended priming is prevented or is reduced to a level that is insignificant. During a heat activation step the chemical modification is removed from the polymerase, thereby regenerating the active enzyme and initiating PCR amplification. The heat activation step generally occurs at a temperature well above the primer annealing temperature. Non-limiting examples of commercially available chemically modified or reversible inactivated thermostable DNA polymerases include Ampli Taq Gold® DNA polymerase, FastStart® Taq DNA polymerase, HotStar® Taq DNA polymerase, Cheetah® Taq DNA polymerase, and Maxima Hot Start® Taq.

In a reversibly-inactivated DNA polymerase, lysine residues can be reversibly blocked by chemical modification of the ε-amino group of the lysine residue. Modification of the lysines in the active region of the protein then results in inactivation of the protein. Additionally, modification of lysines outside the active region may contribute to the inactivation of the protein through steric interaction or conformational changes. A number of compounds have been described that react with amino groups of a protein thereby reversibly modifying the same. For example, amino groups have been reversibly modified by trifluoracetylation (see Goldberger and Anfinsen, 1962, Biochemistry 1:410), amidination (see Hunter and Ludwig, 1962, J. Amber. Chem. Soc. 84:3491), maleylation (see Butler et al., 1967, Biochem. J. 103:78), acetoacetylation (see Marzotto et al., 1967, Biochem. Biophys. Res. Commun. 26:517; and Marzotto et al., 1968, Biochim. Biophys. Acta 154:450), tetrafluorosuccinylation (see Braunitzer et al., 1968, Hoppe-Seyler's Z. Physiol. Chem. 349:265), and citraconylation (see Dixon and Perham, 1968, Biochem. J. 109:312-314; and Habeeb and Atassi, 1970, Biochemistry 9(25):4939-4944).

Further, U.S. Pat. No. 5,773,258 and U.S. Pat. No. 5,677,152 disclose methods of reversibly inactivating thermostable DNA polymerases by reacting a modifier reagent (e.g. dicarboxylic acid anhydrides) with the lysine amino groups of the polymerase. It is disclosed that the covalent lysine modification may be formed by reaction of the DNA polymerase with a modifier reagent. Herein, the modifier reagent may be dicarboxylic acid anhydride. Non-limiting examples of dicarboxylic acid anhydrides useful to reversibly inactivate DNA polymerases include maleic anhydrides; substituted maleic anhydrides such as citraconic anhydride, cis-aconitic anhydride, and 2,3-dimethylmaleic anhydride; exo-cis-3,6-endoxo-Δ⁴-tetrahydropthalic anhydride; and 3,4,5,6-tetrahydrophthalic anhydride. Thus, it is disclosed that the dicarboxylic acid anhydride may be selected from the group consisting of maleic anhydride, citraconic anhydride, cis-aconitic anhydride, 2,3-dimethylmaleic anhydride, exo-cis-3,6-endoxo-Δ4-tetrahydrophthalic anhydride and 3,4,5,6 tetrahydrophthalic anhydride. In some embodiments, the covalent lysine modification is a citraconyl modification. In some embodiments, the covalent lysine modification is an aconitylated modification. In some embodiments, the covalent lysine modification is a 2,3-dimethylmaleated modification. The methods of the present disclosure are not limited to the anhydrides exemplified, but may be applied to any chemical amino acid modification, which is capable to reversibly inactivate a DNA polymerase and can be detected by mass spectrometry. The methods provided herein may be used to detect the position of a chemical modification (e.g. lysine modification) in a DNA polymerase, as well as the extent of a chemical modification (lysine modification) in a DNA polymerase. Where a chemical modification in a DNA polymerase is detected using the methods provided herein including embodiments thereof, the chemical modification is the same for each modified residue detected. For example, where a DNA polymerase is citraconylated, the methods provided herein detect the citraconylated residues present in a DNA polymerase, whereas when a DNA polymerase is aconitylated, the method detects aconitylated residues.

In the methods provided herein including embodiments thereof, chemical modifications of DNA polymerases are detected using mass spectrometry. In some embodiments, mass detection can comprise quadrupole time of flight (Q-TOF) liquid chromatography mass spectrometry (LCMS). Q-TOF/LCMS combines the physical separation capabilities of liquid chromatography with the mass analysis capabilities of mass spectrometry. Using liquid chromatography (LC), sample components including the chemically modified DNA polymerase, are separated by the column depending on their polarity. In general, the less polar and larger the components, the slower they will move through the column. These separation techniques can be performed in an offline or an online fashion. Performing the separation technique in an online fashion, the sample is transferred directly to the mass spectrometer after passing the column and the different components of the sample are analyzed as they elute from the column distributed in time. Performing the separation technique in an offline fashion, the sample is initially separated by the column and the different fractions are stored separately for a later analysis.

Mass Spectrometry generally involves the ionization of the analyte (DNA polymerase or fragments thereof) to generate a charged analyte and measuring the mass of said analyte. During the procedure the sample (e.g., LightCycler eluent buffer or volatile buffer) containing the analyte (DNA polymerase) is loaded onto the MS instrument and undergoes vaporization. The components of the sample are then ionized by one of a variety of methods. For example, during Electrospray-MS (ESI) the analyte is initially dissolved in liquid aerosol droplets. Under the influence of high electromagnetic fields and elevated temperature and/or application of a drying gas the droplets get charged and the liquid matrix evaporates. After all liquid matrix is evaporated the charges remain localized at the analyte molecules that are transferred into the Mass Spectrometer. In matrix assisted laser desorption ionization (MALDI) a mixture of analyte and matrix is irradiated by a laser beam. This results in localized ionization of the matrix material and desorption of analyte and matrix. The ionization of the analyte is believed to happen by charge transfer from the matrix material in the gas phase. For a detailed description of electrospray ionization (ESI) and matrix-assisted laser desorption ionization (MALDI) see e.g. Mano N et al. Anal. Sciences 19 (1) (2003) 3-14. For a description of desorption electrospray ionization (DESI) see Takats Z et al. Science 306 (5695) (2004) 471-473. *See*, *e.g.*, Karas, M.; Hillencamp, F. Anal. Chem. 60:2301 1988); Beavis, R. C. Org. Mass Spec. 27:653 (1992); Creel, H. S. Trends Poly. Sci. 1(11):336 (1993).

The ionized sample components can then be separated according to their mass-to-charge ratio in a mass analyzer. Examples of different mass analyzers used in LC/MS include without limitation single quadrupole, triple quadrupole, ion trap, TOF (time of Flight) and quadrupole-time of flight (Q-TOF). For a detailed description of MS techniques see Mano N et al. Anal. Sciences 19 (1) (2003) 3-14.

In some embodiments, LC-MS detection of covalent lysine-modifications formed by the reaction of lysine residues in a DNA polymerase with a dicarboxylic acid anhydride is performed. The storage and reaction buffer of a chemically modified DNA polymerase has a basic pH of 8-9, which stabilizes the lysine modification and ensures that the reversibly inactivated DNA polymerase remains inactive at temperatures at or below elongation temperature. The lysine modifications are heat and acid labile and can therefore not be separated under the acidic conditions of the mobile phase normally used during standard LC procedures. The inventors have solved this and other problems in the art by performing the LC-process at a modification-stabilizing pH. In some embodiments, the modification stabilizing pH is at least 8. In other embodiments, the modification stabilizing pH is about 8. In other embodiments, the modification stabilizing pH is about 8.2, about 8.4, about 8.6, about 8.8, about 9, about 9.2, about 9.4, about 9.6, about 9.8, about 10, or from about 8-9, 8-10, or 9-10. In some embodiments, the modification stabilizing pH is a polymerase storage buffer pH. A polymerase storage buffer pH refers to the pH of a buffer in which a polymerase is stored. A storage buffer may include polymerase stabilizing components and preservatives (e.g. Tween-20, DTT, glycerol, EDTA) providing for long term storage of the enzyme. In other embodiments, the modification stabilizing pH is a polymerase reaction buffer pH. A polymerase reaction buffer pH refers to the pH of a buffer in which a PCR reaction is performed. A reaction buffer (or reaction mix) may include primers, nucleotides, salts along with stabilizing and preserving components known to be useful for DNA amplification.

While the stabilizing components and preservatives used in DNA polymerase storage and reaction buffers are beneficial for the stability and performance of the DNA polymerase they may be incompatible with mass spectrometry. For instance, the non-ionic detergent Tween-20 helps to stabilize the fairly hydrophobic DNA polymerase, but includes interfering ions and causes ion suppression thereby interfering with the mass spectrometry detection process. Thus, in some embodiments, the methods comprise separating non-ionic detergents from the DNA polymerase prior to mass spectrometric analysis. In other embodiments, the modified polymerase is not stored in the presence of any, or a significant amount, of a non-ionic buffer. Thus, in some embodiments, the DNA polymerase is substantially free of non-ionic detergent immediately prior to and during the detecting of step (i).

The methods provided herein including embodiments thereof can be used to detect the mass of a modified DNA polymerase using LC/Q-TOF mass spectrometry. In some embodiments, the mass of the lysine-modified DNA polymerase is a computer readable mass. A computer readable mass is formed where the mass spectrometry detector is connected to a computer-implemented system, and where the computer-implemented system transforms the electronic signals from the detector into a mass spectrum thereby forming computer readable mass. A computer-implemented system may be used in the methods provided herein to calculate the mass of a DNA polymerase without lysine modifications to form a computer readable standard mass. Thus, in some embodiments the mass of the polymerase without lysine modifications is constructed in silico, thereby forming a computer readable standard mass.

In order to calculate the number of modifications (including but not limited to lysine modifications) in a modified DNA polymerase the mass (e.g., computer readable mass) of the modified DNA polymerase is compared to the mass (e.g. computer readable standard mass) of a polymerase without modifications. By calculating the difference of the mass of the modified DNA polymerase and the mass of the polymerase without modifications and dividing said difference by the mass of the modification itself, the number of modifications attached to the polymerase is determined.

In some embodiments, the calculating is performed on a computer. Accordingly, in some embodiments, "calculating" refers to using a computer-implemented algorithm to calculate number of chemical (lysine) modifications in DNA polymerase. The calculating may be performed by an accurate-mass algorithm to locate a molecular entity or "compound" based on a group of ions (related *m*/*z* values) which are found to be associated with the exact molecular weight. A computer-implemented algorithm may perform the calculation of the difference of the mass of the modified DNA polymerase and the mass of the polymerase without modifications and dividing said difference by the mass of the modification to determine the number of modification(s). In some embodiments, the computer-implemented algorithm is an accurate-mass algorithm. An accurate-mass algorithm locates a molecular entity or "compound" based on a group of ions (related *m*/*z* values) which are found to be associated with the exact molecular weight.

As described above the methods provided herein may be used to detect the extent of a chemical modification (lysine modification) in a DNA polymerase. Further, the methods provided herein may also be used to detect the position of a chemical modification (e.g. lysine modification) in a DNA polymerase. The methods provided herein may be used for the detection of lysine modifications present in an unfragmented DNA polymerase as described above, or in a fragmented DNA polymerase. A fragmented DNA polymerase as provided herein is a polymerase that has been digested with an enzyme (protease) capable of hydrolyzing a protein at a known site(s) within the polymerase. In some embodiments, the enzyme is trypsin.

Where the method includes detecting a fragmented DNA polymerase, the protease digestion can be performed prior to the detection of the mass of the chemically modified (lysine-modified) DNA polymerase. Thus, in some embodiments, the method further includes, prior to the detecting step, fragmenting the modified (e.g., lysine-modified) DNA polymerase with an enzyme that fragments the polymerase at known sites within the polymerase. The mass of the fragments of the modified DNA polymerase is then detected and compared to the mass of potential (predicted) fragments of the polymerase with or without lysine modifications. Thus, in some embodiments, the method further includes detecting the mass-to-charge ratio of fragments of the modified DNA polymerase and comparing the mass of fragments of the modified DNA polymerase to the mass of potential fragments of the polymerase without modifications, thereby determining the location of the modifications at particular sites (e.g., lysines) in the polymerase. In some embodiments, the location of lysine modification corresponds to amino acid position 540, 663, and/or 738 of Taq GOLD polymerase. In other embodiments, the location of lysine modification corresponds to amino acid position 542, 665, and/or 740 of Z05 GOLD polymerase. The term "Gold" indicates that the underlying polymerase (e.g., Taq or Z05 in the examples) has been chemically-modified at lysine residues with a dicarboxylic acid anhydride. The Taq and Z05 polymerase amino acid sequences are known and are described in, for example, US Patent Publication No. 2012/0258501.

It is disclosed that the one or more modification may block the enzyme thereby preventing the formation of one or more fragment and increasing the size of a resulting fragment. For example, where the enzyme is trypsin and the lysine modification is a citraconyl modification, the citraconyl modification may prevent hydrolysis at a citraconylated lysine. Therefore, no cleavage occurs at the citraconylated lysine resulting in a larger size peptide (fragment) compared to the same peptide (fragment) derived from an unmodified DNA polymerase. In some embodiments, the modified DNA polymerase includes two cleavage sites, one of which carries a modification. Therefore, the enzyme cleaves at the unmodified site while the second site remains uncleaved due to the modification. The enzyme cleavage results in the formation of fragment AB and fragment C. The same cleavage of an unmodified DNA polymerase may form a fragment A, a fragment B and a fragment C. The masses of the total number of fragments in the modified DNA polymerase can be calculated by comparing to the masses of fragments A, B, and C in the unmodified DNA polymerase. Therefore, the mass is calculated as the sum of the masses of fragment AB, the mass of the modification in AB and the mass of fragment C. In some embodiments, the calculation is performed by a computer-implemented algorithm.

The methods provided herein can further be used to detect the relative amount of modification at a particular residue (e.g. residue 540, 663, or 738 of Taq GOLD polymerase). The "relative amount" refers to the proportion of a particular amino acid reside at a particular position in the enzyme that is modified. As an example, a polymerase sample could comprise a modified residue at a particular position in 60% of the polymerases in a sample, with the other 40% being unmodified (indicating, for instance, that the polymerase sample is incompletely modified and thus possibly active in spite of the modifications on other copies of the polymerase). A PCR reaction buffer may contain a DNA polymerase, where the relative amount of modification at a particular residue varies among the individual DNA polymerase molecules present in the buffer. The relative amount of modification at a particular residue among these DNA polymerase molecules correlates to the level of chemical inactivation of the individual molecule and therefore provides information regarding the activity of a DNA polymerase in buffer. Thus, in some embodiments, the method includes detecting the relative amount of modification at the amino acid position, wherein the relative amount of modification is the amount of modified lysine residue at the position divided by the sum of the amount of modified and unmodified lysine residue at said position, thereby detecting the relative amount of modification at the position. Such information can be subsequently correlated to predicted polymerase activity (or in activity) and can be used, for instance in quality control when inactivated enzyme is produced.

The methods provided herein may also be used to determine a relative level of chemical modifications at particular residues (e.g. lysine) in a DNA polymerase. Thus, in some embodiments, the method includes detecting the relative amount of fragments of the lysine-modified DNA polymerase and optionally comparing the relative amount of the fragments of the lysine-modified DNA polymerase to a predicted relative amount of potential fragments of the polymerase without lysine modifications, thereby determining the relative level of lysine modifications at particular lysines in the polymerase.

### III. Examples

Provided herein are methods for the analysis of all DNA polymerases compatible with all formulations (i.e. manufacturing retains, stocks, bulks and master mixes). These methods are intended to support all areas in the lifecycle of a DNA polymerase product. The addition of mass spectrometry provides a molecular level characterization which can potentially expand the fundamental understanding of DNA polymerases. As a research tool, the novel data generated from mass spectrometry can be used to elucidate important new information regarding the production, storage and use of chemically modified (GOLD) DNA polymerases. These results provide information which potentially may lead to improving processes and/or performance. Another research application is the analysis of DNA polymerases in master mixes. Studies of this type may be insightful in evaluating environmental effects on the structural integrity of DNA polymerases (i.e. temperature, storage and PCR cycling.) These types of analyses can also play a role in production and trouble-shooting the performance of DNA polymerases. Analysis of DNA polymerases by mass spectrometry would add a novel dimension to assist in Manufacturing, Development and product care. Potential applications for this type of molecular characterization include verification of identity and quantification in all formulations, identification and/or monitoring of protein degradation, characterization of intentional and unintentional modifications and optimization of storage conditions.

The methods provided herein provide information that was previously unattainable, i.e., primary sequence determination, structural characterization, identification of point mutations and micro-heterogeneity. Addition of this technology to the molecular toolbox enables the ability to confirm identity, identify modifications, mutations and/or cleavages, monitor stability at a molecular level and assist in developing novel DNA polymerases.

Mass spectrometry has become a useful and widely adopted tool in the study of proteins due to its exquisitely high sensitivity and accuracy. The applications for protein analysis include molecular weight determination, identification, quantitation and sequencing (1-8). It is also used for studying post translational modifications, protein structure and non-covalent interactions (9-13). These applications are all within the capacity of a QTOF LCMS instrument. The data obtained from mass spectrometry provide a new pathway into the characterization of DNA polymerases. In the feasibility work, methods were developed for the intact analysis of truncated DNA polymerases. This provides new methods to quickly identify and differentiate closely related molecules i.e. Stoffel Fragment and delta Z05 DNA polymerases. In addition, these methods may also be used to identify degradation, modification and/or cleavage. Methods of this type can play a role in problem solving issues regarding the manufacture and use of DNA polymerases.

Until recently, the ability to examine the heterogeneities in DNA polymerase, and particularly in chemically modified (GOLD) enzymes, has been very limited. Recently LCMS methods were developed that were able to (1) characterize the positions of the enabling chemical modifications made to these molecules and (2) assess alterations to the protein at the molecular level. This tool enables the initiation of fundamental studies into the GOLD enzymes including local variation of the chemical modifications, stability, reactivation at a molecular level and reactivation differences between Taq Gold and Z05 Gold DNA polymerases.

The final destination for most DNA polymerases is a PCR master mix, ultimately in a kit. The typical concentration may be 25-100 nM which is prohibitively beyond the capabilities of many standard biochemical characterization techniques. The currently available characterization methods are the DNA polymerase activity assay and silver stain PAGE. Both methods are quite sensitive but are prone to some amount of method variation and are not routinely performed. The QTOF LCMS has been reported as having a limit of protein quantitation in the high fmoles but this is highly compound dependent. Provided herein are methods for the analysis of DNA polymerase in a master mix and to evaluate their usefulness for both qualitative and quantitative analysis. These methods enable the ability to both accurately quantitate the enzyme concentration and to evaluate its structural integrity in master mix. This tool is useful to verify the enzyme identification and concentration. It may also be used to study the effects of temperature, storage and PCR on the structural integrity of DNA polymerases.

Polymerases such as GOLD DNA polymerases are reversibly inactivated by covalently derivatizing lysine residues with citraconic anhydride. Until recently there were no methods capable of determining how many, or which, of the many lysine residues are actually derivatized. The present example uses a LCMS QTOF method capable of identifying derivatized lysine residues in GOLD DNA polymerases. This technology enables the full characterization of GOLD DNA polymerases at a molecular level not attainable in any other way. This enabling technology serves to fill in gaps in the fundamental knowledge of these molecules including without limitation mapping of derivatized residues; studying reactivation kinetics as a function of derivatization at the residue level; gaining a molecular understanding of the hot start mechanism; determining lot to lot variability; providing a possible QC method; optimizing the gilding process; potentially engineering improved GOLD DNA polymerases; studying and/or troubleshooting stability; and evaluating the performance of partially reactivated GOLD DNA polymerases.

The high resolution and mass accuracy afforded by the Agilent QTOF LCMS enables these types of analyses. The QTOF is capable of 300-fold better mass accuracy and a 20-fold improvement in resolution in comparison to the single quadrupole and ion trap mass analyzers in the CIT department. The combined high mass accuracy and high resolution minimizes spectral interference from excipient molecules and enables mass assignment to the 4^{th} decimal place.

The Agilent mass spectrometer is composed of a UPLC, thermostated auto sampler and column compartments, a diode array UV detector, an electrospray ionization (ESI) source and a quadrupole time-of-flight (QTOF) mass analyzer. The ESI QTOF combination offers very high sensitivity, resolution and accuracy which are used to unequivocally deduce the elemental composition of compounds. The quadrupole can be used as a simple scanning analyzer or to selectively isolate a precursor ion and direct that ion into the collision cell for fragmentation. The TOF component has an upper mass-to-charge ratio *(m*/*z)* in excess of 10,000 and a high resolving power (10,000-15,000). The quadrupole-TOF instrument can be used as either a single quadrupole or tandem MS experiments. Due to its high accuracy and sensitivity, the ESI QTOF mass spectrometer is being used for the analysis of proteins in the fields of proteomics and pharmacokinetics (14). The methods described herein are directed towards utilizing this powerful tool for the analysis of DNA polymerases.

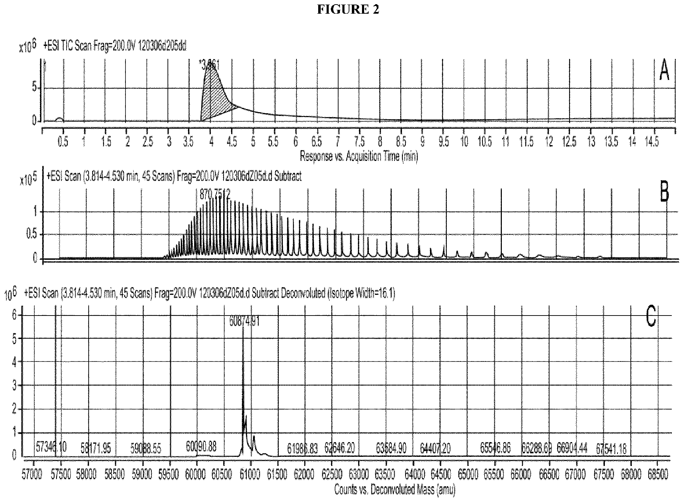

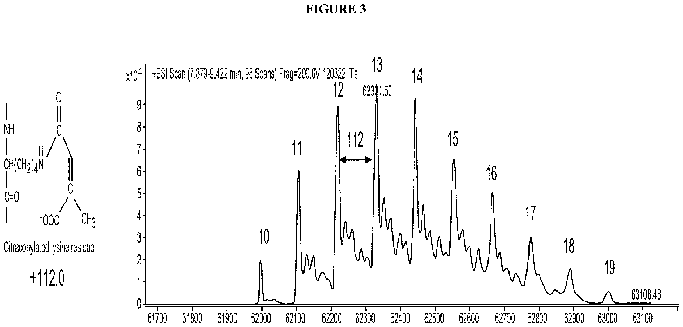

Methods have been developed for the intact analysis of delta Z05, Stoffel Fragment and the GOLD (chemically-modified) versions of these. These methods provide the ability to quickly determine accurate mass and also some structural data. In the analysis of delta Z05, the data confirms identity and also shows that the N-terminal methionine and alanine residues are cleaved (Figure 2). The analysis of the GOLD DNA polymerases provides structural information that was previously unobtainable (Figure 3). The GOLD DNA polymerases are produced by a reversible modification of lysine residues with citraconic anhydride. Prior to this work, there was no knowledge as to the extent or location of these modified lysines. Using novel methods developed to preserve the heat and acid labile citraconyl modification, it is now possible to determine their global distribution in delta Z05 GOLD (Figure 3). This technology provides a tool to more fully characterize these molecules and to initiate studies into their manufacture, stability and storage.

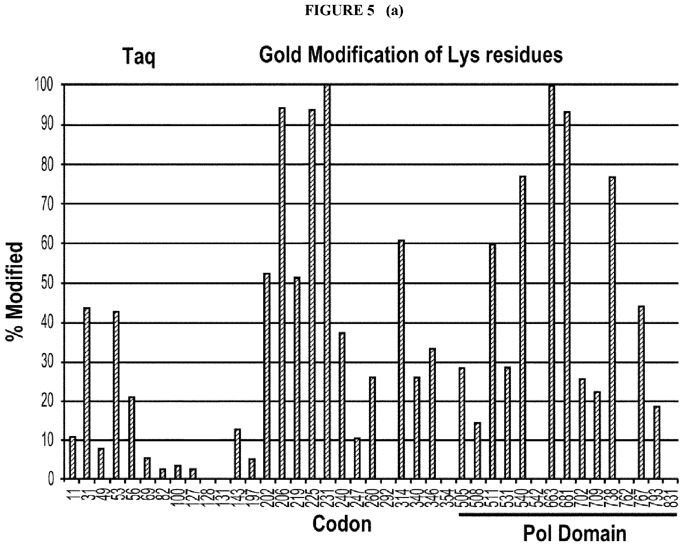

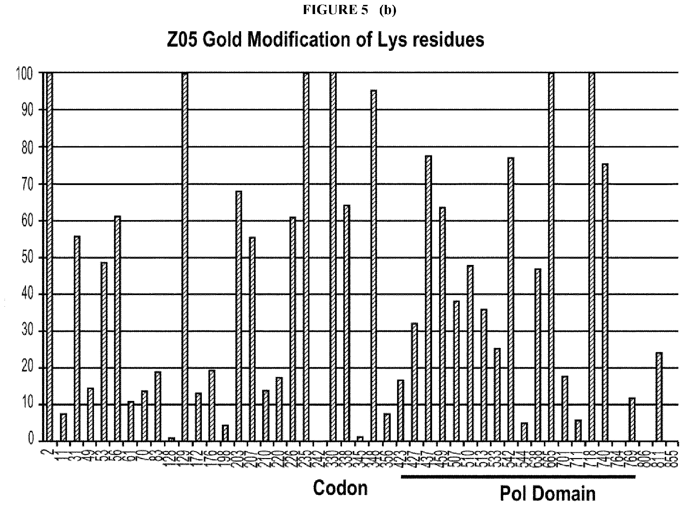

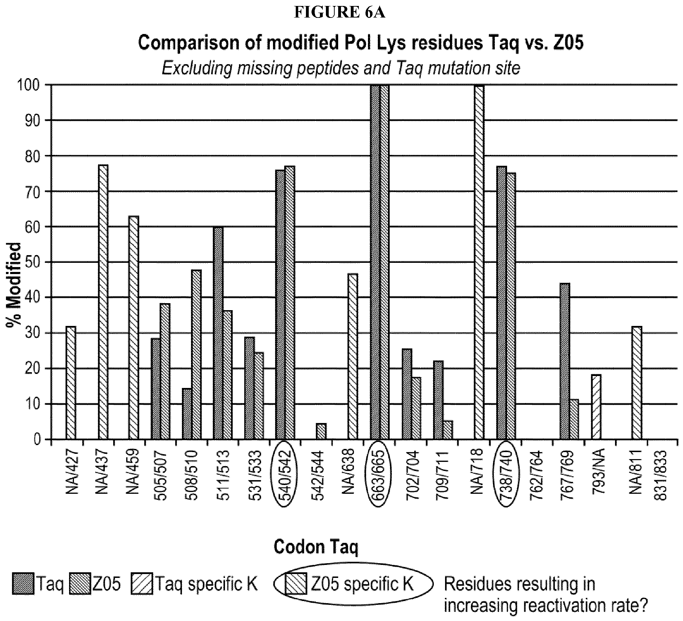

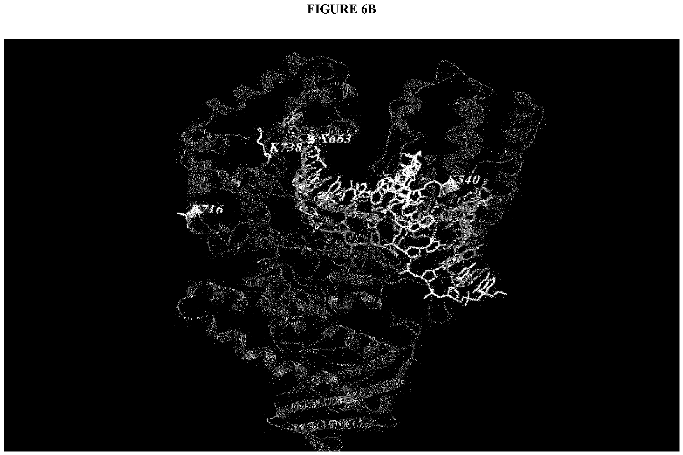

Methods have also been developed for the peptide sequencing of Z05 GOLD and Taq GOLD DNA polymerases. This data provided the first detailed map of the chemical modifications made to these molecules. The analysis revealed that the degree of modification of individual lysine residues ranged from 0 to 100%, and that homologous lysines in the two DNA polymerases tended to have similar degrees of modifications (Figure 5). Comparing modifications in the DNA polymerase domain revealed that three positions are highly modified in both Taq GOLD and Z05 GOLD (K540, K663 and K738 in Taq). Analyzing these positions in a molecular model indicates that all three positions are actively involved in the replication process (Figure 6). The detailed information revealed in these studies demonstrates the power of mass spectrometry as an analytical tool. In some embodiments, mass spectrometry is used for the analysis of DNA polymerases in their native buffers and concentrations.

### Material and Methods

The ability to analyze DNA polymerases is related to developing sample preparation methods to remove interfering components. For example, the detergent Tween 20 is incompatible with mass spectrometry because it has interfering ions and can also cause ion suppression. This detergent is present in all enzyme storage buffers and PCR master mixes to help stabilize the fairly hydrophobic DNA polymerases. Ideally the sample preparation method would be independent of the buffer pH, the DNA polymerase concentration, and the DNA polymerase size (i.e. intact or digested). The method would concentrate the sample, have high recoveries with no preferential protein binding and remove the vast majority of the Tween 20. Such a method could be universally applied to all DNA polymerases, in all formulations (stocks, bulks, master mixes) and either intact protein or tryptic digests.

### Develop sample preparation methods for tryptic digests.

The tryptic reaction is typically very dilute with an acidic pH. Two methods have been recommended (1) a spin column from Pierce and (2) a strong cation exchange (SCX) resin packed in a pipet tip. In the first, the Tween 20 is bound to the support and the peptides pass through. The second retains the peptides, followed by a wash to remove the Tween 20 and then elution of the peptides. The residual Tween 20 in the eluted peptides will be quantitated by ELSD. The protein recovery will be determined by comparison to an unprocessed control digest.

### Develop LC-MS/MS methods for peptide mapping.

Peptide mapping is accomplished through an overnight tryptic digest of the protein followed by analysis on the QTOF LCMS. Methods include 1) a chromatography method to separate the peptides and 2) the identification of source conditions for the mass spectrometer. This resulted in a high degree of sequence coverage. The addition of tandem MS would serve to verify the peptide sequences and thus improve the reliability of the overall sequence data. The process of tandem MS involves the selection of ions (precursors) for fragmentation and identification of parameters (collision energy) to efficiently fragment it.

### Develop sample preparation methods for GOLD DNA polymerases.

The GOLD DNA polymerases are stored in a high pH buffer in order to preserve the acid and heat labile citraconyl modifications. Maintaining the integrity of these modifications with a high pH buffer is essential. This pH is well above the DNA polymerase pI and thus the DNA polymerase will have a negative net charge which makes them suitable for anion exchange solid phase extraction. The DNA polymerases are known to not be retained on strong anion exchange (SAX) at pH 8. The retention of both intact and digested DNA polymerases will be evaluated at pH's in the range of 10 and 11. The residual Tween 20 will be quantitated by ELSD. Protein recovery will be evaluated by PAGE for the intact DNA polymerase and by comparison to the LCMS profile of a non-treated control for the digests. Alternatively the use of precipitation of high concentrated stocks will be evaluated. This will be determined by comparison to the LCMS profile of an un-precipitated control. The digest would be performed on the resuspended protein.

### Develop LC-MS/MS methods compatible with tryptic digests of chemically modified DNA polymerases.

The methods for the separation of tryptic digests typically employ acidic buffers and temperatures above ambient. Methods were previously developed for these molecules using basic buffers run at 20 °C. These methods are less sensitive than the acid method due to a decrease in ionization efficiency at high pH. Despite these issues the sequence coverage maps were above 90%. The addition of tandem MS would serve to clarify conflicting results and verify the peptide sequences to improve the reliability of the overall sequence data..

### Study hydrolysis of citraconyl modifications during reactivation.

The development of the above methods will enable studies into the reactivation process at a molecular level. These studies can be accomplished by performing partial reactivation of the GOLD DNA polymerases in PCR buffer, determining the resultant activity, and mapping the modifications by peptide sequencing

### Identification and characterization of DNA polymerases in PCR master mixes.

These methods are intended to serve as a support tool to quickly confirm the DNA polymerase identity and quantity. In addition they can be used to detect structural issues such as cleavage or oxidative damage.

### Develop sample preparation methods for PCR master mix.

PCR master mixes contain many constituents that may adversely affect the recovery and/or efficiency of the methods developed herein. Sample preparation of a PCR master mix has an additional requirement of maintaining or increasing the sample concentration to ≥ 40nM. The approach is to repeat the methods development processes outlined above with DNA polymerases in representative PCR master mixes as appropriate.

### Qualitative analysis by LCMS.

The concentration of DNA polymerases in master mix is relatively low, which will result in the injection of less sample in a higher volume. The methods developed herein will be evaluated by 1) determining the limit of detection (LOD) and 2) studying the effect of increasing the sample volume.

### Quantitation by LCMS.

The feasibility of quantitating the DNA polymerase was evaluated by the development of a reference standard. The approach was to evaluate the method reported by Silva et al (15) which describes a method for absolute protein quantitation from tryptic digests. They discovered that there is a relationship between the MS signal response and the protein concentration. In particular, the average MS signal response from the three most intense tryptic peptides per mole of proteins is constant within a coefficient of variation of less than ±10%.

LCMS QTOF was successfully used to fully characterize model Gold DNA polymerases by characterizing the level of derivatization for all but 2 of the lysine residues. This work was performed using polymerases that were derivatized in the absence of Tween 20. The majority of lysine residues in both Z05 GOLD and Taq FS E681K GOLD were found to be derivatized to some extent, ranging from 2-100%. Very few residues were found to be 100% derivatized. Of particular interest was that 3 residues in the polymerase domain were found to be heavily modified in both Taq and Z05 (K540/542, K663/665 and K738/740 respectively.) Modeling results indicated that K663/665 and K540/542 are in the catalytic site and K738/740 interacts with the template DNA. This suggests that the derivatization of these residues is likely involved in inactivation of the polymerase. The K663/665 residue in particular was found to be 100% derivatized and is reported in the literature as being immutable. These types of comparisons offer a new tool for understanding Gold polymerases at a molecular level.

**Table 1: Summary of derivatized lysine residues identified by LCMS QTOF**

| **Average derivatization** - **excluding missing peptides and Taq mutation** (**K681**) | | | | |
|---|---|---|---|---|
| *Taq FS E681K* (-) *K100, K171, K681, K804* | | | | |
| *Z05* (-) *K101, K172, K324, K806* | | | | |
| | # K | #K observed | % Mod (Avg) | Avg #CIT/ Molecule |
| Taq FS E681K | 43 | 39 | 31.6 | 12.3 |
| Z05 | 49 | 45 | 40.7 | 18.3 |
| Taq FS E681K (Pol only) | 16 | 14 | 35.2 | 4.9 |
| Z05 (Pol only) | 20 | 18 | 43.3 | 7.8 |

### Chemicals

Citraconic anhydride, dimethyl formamide and ammonium bicarbonate were purchased from Sigma. Sequencing grade trypsin was purchased from Promega. Ammonium hydroxide and acetonitrile were purchased from VWR.

### Preparation of Gold DNA polymerase

The DNA polymerases are reacted with citraconic anhydride at 4°C overnight. The derivatized DNA polymerase are buffer exchanged into a standard protein storage buffer.

### Digestion of Gold DNA polymerase

The Gold DNA polymerase is digested at 37°C overnight with trypsin using standard procedures outlined in the protease package insert. The digested polymerase is diluted to 1µM by dilution with ammonium hydroxide.

### LC/MS conditions

A 5 pmole aliquot was analyzed on a Zorbax Extend C-18 2.1x100mm, 3.5µm column installed on an Agilent 1200 LC. The column temperature was 35°C. The A mobile phase was 20mM ammonium hydroxide and the B mobile phase was 20mM ammonium hydroxide in acetonitrile. A linear gradient of 5-50%B over 45 minutes was run at a flow rate of 0.2ml/min. The column was washed with 100%B for 5 minutes and then re-equilibrated for 15 minutes. A standard dual electrospray source was installed on an Agilent 6530 QTOF. The drying gas temperature was 350°C with a flow of 12 l/min. The Nebulizer was 30psig, the Fragmentor voltage was 180V and the Capillary voltage was 4000V. The data was acquired in scan mode (*m*/*z* 1400-3200) in positive ion.

### Data Analysis

Compounds were identified using the Find by Molecular Feature tool in the Agilent Mass Hunter software. The parent protein sequence was digested in-silico and the theoretical peptides were matched against the identified compounds. The citraconyl group was added as a predicted modification and 2 missed cleavages were allowed. The data was interpreted as follows:
1. For every lysine residue there is likely to be a modified and unmodified species. The presence of a citraconyl modification will typically inhibit trypsin cleavage. The unmodified lysines will thus typically be on the C-terminus of a peptide.
2. The % modification at each lysine is calculated by dividing the modified volume by the sum of the volumes for modified and unmodified (see Table 2
3. Some peptides will have more than 1 lysine and require some user interpretation. Group all peptides associated with each lysine residue, replicating lines with multiple lysines. Determine the modification at the distinct codon for each peptide in the group. Total the volume of modified and unmodified for each particular codon and calculate the % modified (see Table 3).

**Table 2. Calculation of % modified lysine in a peptide with a single lysine.**

| Volume modified / Total volume of modified and unmodified (1189698 / (1189698+4820758) = 19.8% | | | | | |
|---|---|---|---|---|---|
| **Seq Loc** | **Sequence** | **Missed** | **Pred Mod** | **Volume** | **SEQ ID NO:** |
| A(705-709) | AWIEK | 0 | 0 | 4820758 | 1 |
| A(705-715) | AWIEKTLEEGR | 1 | 1 | 1189698 | 2 |

**Table 3. Calculation of % modified lysine in a peptide with multiple lysines.**

| **K Codon** | **Seq Loc** | **Sequence** | **Pred Mod** | **Volume** | **Modified** | **SEQ ID NO:** |
|---|---|---|---|---|---|---|
| 505 | A(493-505) | VLFDELGLPAIG**K** | 0 | 10505448 | N | 3 |
| 505 | A(493-508) | VLFDELGLPAIG**K**TE**K** | 1 | 3621938 | Y | 4 |
| 505 | A(493-511) | VLFDELGLPAIG**K**TE**K**TG**K** | 2 | 471858 | Y | 5 |
| | | | | | | |
| 508 | A(493-508) | VLFDELGLPAIG**K**TE**K** | 1 | 3621938 | N | 4 |
| 508 | A(506-508) | TE**K** | 0 | 337397 | N | - |
| 508 | A(493-511) | VLFDELGLPAIG**K**TE**K**TG**K** | 2 | 471858 | Y | 5 |
| 508 | A(506-511) | TE**K**TG**K** | 1 | 292805 | Y | 6 |
| 508 | A(506-512) | TE**K**TG**K**R | 2 | 437454 | Y | 7 |
| | | | | | | |
| 511 | A(493-511) | VLFDELGLPAIG**K**TE**K**TG**K** | 2 | 471858 | N | 5 |
| 511 | A(506-511) | TE**K**TG**K** | 1 | 292805 | N | 6 |
| 511 | A(506-512) | TE**K**TG**K**R | 2 | 437454 | Y | 7 |

| **K Codon** | **Total Modified** | **Total Unmodified** | **% Modified** |
|---|---|---|---|
| 505 | 4093796 | 10505448 | 28% |
| 508 | 1202117 | 3959335 | 23% |
| 511 | 437454 | 764663 | 36% |

### High pH method for digested GOLD polymerase

Because citraconyl modifications are heat and acid labile, a high pH method was used to ensure integrity of citraconyl modifications. This method was developed for analysis of Taq GOLD and Z05 GOLD in atypical storage buffer (20mM Tris pH 8, no KCl or Tween 20). Standard GOLD storage buffer is: 20mM Tris pH 9, 0.1 mM EDTA, 1 mM DTT, 50 % (v/v) Glycerol, 0.2% Tween 20.

Tryptic digests were carried out overnight in NH₄CO₃ buffer (∼pH 8) at 37°C. The amount of sample analyzed in previous studies was around 5 pmole. Polymerase concentration was around 15µM during digestion. After completion, digested samples were diluted to around 1µM with addition of Tris pH 9 to keep pH high. 5µl of the digested sample was analyzed by LCMS.

Sample handling: The pH of GOLD polymerase digests were adjusted up to pH 9 upon completion of digest. For LCMS analysis, the sample was diluted to 1µM in NH₄OH (Buffer A), filtered, and 5µl (5 pmoles) was analyzed.

### LCMS Method dZ05 Gold

Method name: ESI_ProteinDigest_5_50. Buffer A1: 20mM NH4OH; Buffer B1: 20mM NH₄OH in CH₃CN; Flow: 0.2ml/min; Acquisition: UV A215nm; Column: Zorbax Extend C18 (2.1x100mm) p/n 761753-902; Temp: 35°C; Gradient: min %B: 0 5; 45 50; 46 95; 50 95; Post time: 15 minutes; Source: Dual ESI; Mode: Positive; Range:100-1700; Gas: 350°C; 12 L/min; Nebulizer: 30 psig; V Cap: 4000V; V Frag: 180 V.

### Data Analysis

A MFE (Molecular Feature Extraction) algorithm was run, sequence was loaded into the sequence editor; and citraconyl (112.016045) was added to the chemical dictionary (C₅O₃H₅). Total mass reflected the addition of citraconyl group(s) and loss of 1H from the peptide, allowing two missed cleavages. Citraconyl was added to predict modifications. The match sequence was run against in-silico digested sequence.

For every lysine residue there is likely to be a modified and unmodified species. Presence of citraconyl modification typically inhibits trypsin cleavage. Unmodified lysines will thus be usually located on the C-terminus of the peptide. The % modification was calculated for each lysine as follows: Volume modified / Total volume of modified and unmodified (1189698 / (1189698+4820758) = 19.8% (Table 2).

Some peptides will have more than 1 lysine and require some user interpretation. Group all peptides associated with each lysine residue, replicating lines with multiple lysines. Determine the modification at the distinct codon for each peptide in the group. Total the volume of modified and unmodified for each particular codon and calculate % modified.

It is understood that the examples and embodiments described herein are for illustrative purposes only and that various modifications or changes in light thereof will be suggested to persons skilled in the art.

## Claims

1. A method of detecting the number of covalent lysine modifications in a lysine-modified DNA polymerase, said method comprising
(i) detecting the mass of the lysine-modified DNA polymerase at a modification stabilizing pH of at least 8; and
(ii) calculating the number of lysine modifications in the lysine-modified DNA polymerase by comparing the mass of the lysine-modified DNA polymerase to a polymerase without lysine modifications, wherein the number of lysine modifications is the difference of the mass of the lysine-modified DNA polymerase and the polymerase without lysine modifications divided by the mass of the lysine modification, thereby detecting the number of lysine modifications in the DNA polymerase,
wherein said covalent lysine modification is a citraconyl modification, an aconitylated modification or a 2,3-dimethylmaleated modification..

2. The method of claim 1, wherein said mass of the lysine-modified DNA polymerase is a computer readable mass.

3. The method of claim 2, wherein said mass of the polymerase without lysine modifications is constructed in silico, thereby forming a computer readable standard mass.

4. The method of claim 3, wherein said calculating is performed on a computer.

5. The method of any one of claims 1 to 4, wherein said DNA polymerase is substantially free of non-ionic detergent immediately prior to and during said detecting of step (i).

6. The method of any one of claims 1 to 5, further comprising, prior to the detecting of step (i), fragmenting the lysine-modified DNA polymerase with an enzyme that fragments the polymerase at known sites within the polymerase.

7. The method of claim 6, wherein the enzyme is trypsin.

8. The method of claim 6, further comprising detecting the mass-to-charge ratio of fragments of the lysine-modified DNA polymerase and comparing the mass of fragments of the lysine-modified DNA polymerase to the mass of potential fragments of the polymerase without lysine modifications, thereby determining the location of lysine modifications at particular lysines in the polymerase.

9. The method of claim 8 further comprising detecting the relative amount of fragments of the lysine-modified DNA polymerase and optionally comparing the relative amount of the fragments of the lysine-modified DNA polymerase to a predicted relative amount of potential fragments of the polymerase without lysine modifications, thereby determining the relative level of lysine modifications at particular lysines in the polymerase.

10. The method of claim 8, wherein said location of lysine modification corresponds to amino acid position 540, 663, or 738 of a Taq GOLD DNA polymerase.

11. The method of claim 8, wherein said location of lysine modification corresponds to amino acid position 542, 665, or 740 of a Z05 GOLD DNA polymerase.

12. The method of claim of 11, comprising detecting the relative amount of modification at said amino acid position, wherein the relative amount of modification is the amount of modified lysine residue at said position divided by the sum of the amount of modified and unmodified lysine residue at said position, thereby detecting said relative amount of modification at said position.

## Patentansprüche

1. Verfahren zum Detektieren der Zahl von kovalenten Lysin-Modifizierungen in einer Lysin-modifizierten DNA-Polymerase, wobei das Verfahren Folgendes umfasst:
(i) das Detektieren der Masse der Lysin-modifizierten DNA-Polymerase bei einem Modifizierung-stabilisierenden pH-Wert von mindestens 8; und
(ii) das Berechnen der Zahl von Lysin-Modifizierungen in der Lysin-modifizierten DNA-Polymerase, indem die Masse der Lysin-modifizierten DNA-Polymerase mit einer Polymerase ohne Lysin-Modifizierungen verglichen wird, wobei die Zahl von Lysin-Modifizierungen der Unterschied der Masse der Lysin-modifizierten DNA-Polymerase und der Polymerase ohne Lysin-Modifizierungen, geteilt durch die Masse der Lysin-Modifizierung, ist, wodurch die Zahl von Lysin-Modifizierungen in der DNA-Polymerase detektiert wird,
wobei die kovalente Lysin-Modifizierung eine Citraconyl-Modifizierung, eine aconitylierte Modifizierung oder eine 2,3-Dimethylmaleat-Modifizierung ist.

2. Verfahren nach Anspruch 1, wobei die Masse der Lysin-modifizierten DNA-Polymerase eine computerlesbare Masse ist.

3. Verfahren nach Anspruch 2, wobei die Masse der Polymerase ohne Lysin-Modifizierungen *in silico* hergestellt wird, wodurch eine computerlesbare Standardmasse gebildet wird.

4. Verfahren nach Anspruch 3, wobei das Berechnen auf einem Computer durchgeführt wird.

5. Verfahren nach einem der Ansprüche 1 bis 4, wobei die DNA-Polymerase unmittelbar vor und während des Detektierens in Schritt (i) im Wesentlichen frei von nicht-ionischem Detergens ist.

6. Verfahren nach einem der Ansprüche 1 bis 5, ferner umfassend, vor dem Detektieren in Schritt (i), das Fragmentieren der Lysin-modifizierten DNA-Polymerase mit einem Enzym, das die Polymerase an bekannten Stellen innerhalb der Polymerase fragmentiert.

7. Verfahren nach Anspruch 6, wobei das Enzym Trypsin ist.

8. Verfahren nach Anspruch 6, ferner umfassend das Detektieren des Masse-zuLadung-Verhältnisses von Fragmenten der Lysin-modifizierten DNA-Polymerase und das Vergleichen der Masse von Fragmenten der Lysin-modifizierten DNA-Polymerase mit der Masse von potenziellen Fragmenten der Polymerase ohne Lysin-Modifizierungen, wodurch die Stelle von Lysin-Modifizierungen in bestimmten Lysinen in der Polymerase bestimmt wird.

9. Verfahren nach Anspruch 8, ferner umfassend das Detektieren der relativen Menge von Fragmenten der Lysin-modifizierten DNA-Polymerase und gegebenenfalls das Vergleichen der relativen Menge der Fragmente der Lysin-modifizierten DNA-Polymerase mit einer vorhergesagten relativen Menge von potenziellen Fragmenten der Polymerase ohne Lysin-Modifizierungen, wodurch der relative Grad an Lysin-Modifizierungen in bestimmten Lysinen in der Polymerase bestimmt wird.

10. Verfahren nach Anspruch 8, wobei die Stelle der Lysin-Modifizierung der Aminosäureposition 540, 663 oder 738 einer Taq GOLD DNA-Polymerase entspricht.

11. Verfahren nach Anspruch 8, wobei die Stelle der Lysin-Modifizierung der Aminosäureposition 542, 665 oder 740 einer Z05 GOLD DNA-Polymerase entspricht.

12. Verfahren nach Anspruch 11, umfassend das Detektieren der relativen Menge von Modifizierung an der Aminosäureposition, wobei die relative Menge von Modifizierung die Menge von modifiziertem Lysin-Rest an der Position, geteilt durch die Summe der Menge von modifiziertem und nicht-modifiziertem Lysin-Rest an der Position, ist, wodurch die relative Menge von Modifizierung an der Position detektiert wird.

## Revendications

1. Méthode de détection du nombre de modifications de lysine covalente dans une ADN polymérase à lysine modifiée, ladite méthode comprenant
(i) la détection de la masse de l'ADN polymérase à lysine modifiée à un pH de stabilisation de modification supérieur ou égal à 8 ; et
(ii) le calcul du nombre de modifications de lysine dans l'ADN polymérase à lysine modifiée en comparant la masse de l'ADN polymérase à lysine modifiée à une polymérase sans modifications de lysine, le nombre de modifications de lysine étant la différence de la masse de l'ADN polymérase à lysine modifiée et de la polymérase sans modifications de lysine divisée par la masse de la modification de lysine, ce qui permet de détecter le nombre de modifications de lysine dans l'ADN polymérase,
ladite modification de lysine covalente étant une modification de citraconyle, une modification aconitylée ou une modification 2,3-diméthylmaléatée.

2. Méthode selon la revendication 1, dans laquelle ladite masse de l'ADN polymérase à lysine modifiée est une masse lisible par ordinateur.

3. Méthode selon la revendication 2, dans laquelle ladite masse de la polymérase sans modifications de lysine est construite in silico, ce qui permet de former une masse standard lisible par ordinateur.

4. Méthode selon la revendication 3, dans laquelle ledit calcul est effectué sur un ordinateur.

5. Méthode selon l'une quelconque des revendications 1 à 4, dans laquelle ladite ADN polymérase est pratiquement exempte de détergent non ionique immédiatement avant et pendant ladite détection de l'étape (i).

6. Méthode selon l'une quelconque des revendications 1 à 5, comprenant en outre, avant la détection de l'étape (i), la fragmentation de l'ADN polymérase à lysine modifiée avec une enzyme qui fragmente la polymérase au niveau de sites connus au sein de la polymérase.

7. Méthode selon la revendication 6, dans laquelle l'enzyme est la trypsine.

8. Méthode selon la revendication 6, comprenant en outre la détection du rapport masse sur charge des fragments de l'ADN polymérase à lysine modifiée et la comparaison de la masse des fragments de l'ADN polymérase à lysine modifiée à la masse des fragments potentiels de la polymérase sans modifications de lysine, ce qui permet de déterminer l'emplacement des modifications de lysine au niveau de lysines particulières dans la polymérase.

9. Méthode selon la revendication 8 comprenant en outre la détection de la quantité relative de fragments de l'ADN polymérase à lysine modifiée et éventuellement la comparaison de la quantité relative des fragments de l'ADN polymérase à lysine modifiée à une quantité relative prédite de fragments potentiels de la polymérase sans modifications de lysine, ce qui permet de déterminer le taux relatif des modifications de lysine au niveau de lysines particulières dans la polymérase.

10. Méthode selon la revendication 8, dans laquelle ledit emplacement de la modification de lysine correspond à la position d'acide aminé 540, 663 ou 738 d'une ADN polymérase Taq GOLD.

11. Méthode selon la revendication 8, dans laquelle ledit emplacement de la modification de lysine correspond à la position d'acide aminé 542, 665 ou 740 d'une ADN polymérase Z05 GOLD.

12. Méthode selon la revendication 11, comprenant la détection de la quantité relative de modification au niveau de ladite position d'acide aminé, dans laquelle la quantité relative de modification est la quantité de résidus lysine modifiés au niveau de ladite position divisée par la somme de la quantité de résidus lysine modifiés et non modifiés au niveau de ladite position, ce qui permet de détecter ladite quantité relative de modification au niveau de ladite position.
